(19) Europäisches Patentamt
European Patent Office
Office européen des brevets

(11) **EP 4 031 005 B1**

(12) **FASCICULE DE BREVET EUROPEEN**

(45) Date de publication et mention
de la délivrance du brevet:
**06.03.2024 Bulletin 2024/10**

(21) Numéro de dépôt: **20776241.0**

(22) Date de dépôt: **21.09.2020**

(51) Classification Internationale des Brevets (IPC):
**A61B 5/16** $^{(2006.01)}$    **A61B 5/0205** $^{(2006.01)}$
**A61B 5/053** $^{(2021.01)}$    **A61B 5/024** $^{(2006.01)}$
**A61B 5/00** $^{(2006.01)}$

(52) Classification Coopérative des Brevets (CPC):
**A61B 5/02055; A61B 5/165;** A61B 5/02416;
A61B 5/0533; A61B 5/4035

(86) Numéro de dépôt international:
**PCT/IB2020/058762**

(87) Numéro de publication internationale:
**WO 2021/053632 (25.03.2021 Gazette 2021/12)**

(54) **SYSTÈME DE DÉTERMINATION D'UNE ÉMOTION D'UN UTILISATEUR**

SYSTEM ZUM BESTIMMEN EINER BEWEGUNG EINES BENUTZERS

SYSTEM FOR DETERMINING AN EMOTION OF A USER

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **20.09.2019 FR 1910395**

(43) Date de publication de la demande:
**27.07.2022 Bulletin 2022/30**

(73) Titulaire: **Ovomind K.K.
Tokyo
170-0001 (JP)**

(72) Inventeur: **FRACHI, Yann
13012 Marseille (FR)**

(74) Mandataire: **IP Trust
2, rue de Clichy
75009 Paris (FR)**

(56) Documents cités:
KR-A- 20160 085 577    US-A1- 2008 214 903
US-A1- 2016 345 060    US-A1- 2019 239 795

• DOMÍNGUEZ-JIMÉNEZ J A ET AL: "A machine learning model for emotion recognition from physiological signals", BIOMEDICAL SIGNAL PROCESSING AND CONTROL, ELSEVIER, AMSTERDAM, NL, vol. 55, 3 septembre 2019 (2019-09-03), XP085914451, ISSN: 1746-8094, DOI: 10.1016/J.BSPC.2019.101646 [extrait le 2019-09-03]
• GAUTAM ARVIND ET AL: "A Data Driven Empirical Iterative Algorithm for GSR Signal Pre-Processing", 2018 26TH EUROPEAN SIGNAL PROCESSING CONFERENCE (EUSIPCO), EURASIP, 3 septembre 2018 (2018-09-03), pages 1162-1166, XP033461610, DOI: 10.23919/EUSIPCO.2018.8553191

EP 4 031 005 B1

**Description**

La présente invention concerne le domaine de la détection d'activités physiologiques d'un individu, notamment pour la captation, l'identification et l'analyse des émotions de cet individu afin de permettre une caractérisation automatique par exemple en vue d'une amplification d'émotions chez une personne, ou pour interagir avec un système externe interactif fournissant une expérience immersive pour un utilisateur. A cet effet, on sait exploiter des signaux électrophysiologiques acquis par des capteurs cutanés pour déduire des corrélations avec l'état émotionnel.

Il est connu depuis longtemps que la conductance cutanée ou électrodermale (par exemple) peut être fonction des émotions d'un individu (ex. le polygraphe ou « détecteur de mensonges »). Si ces dispositifs permettent d'indiquer que la conductance cutanée varie, ils ne peuvent pas déterminer simplement le type d'émotions (ou la valence) associées à ladite variation : ladite variation est-elle associée à une grande joie (ou plus généralement associée à une émotion positive) ou à une peur (ou plus généralement associée à une émotion négative) ?

Les travaux de recherche dans le domaine de l'étude des émotions se situent dans deux grands domaines :

- Pour certains chercheurs, dont les travaux se concentrent sur des aspects dimensionnels, les états émotionnels sont régis par des facteurs sous-jacents comme la valence, l'excitation et l'état inotivationnel.

- Pour d'autres chercheurs, dont les travaux se concentrent l'analyse discrète des émotions, chaque émotion a des corollaires expérimentaux, physiologiques et comportementaux.

Jusqu'à présent, cinq méthodes coexistent :
La première méthode de capture des émotions est l'auto déclaration (ou « self-report » en anglais) : cette méthode propose que les émotions soient mesurées et évaluées par la personne elle-même.

La deuxième méthode utilise des outils de mesure des réponses du système nerveux autonome (SNA). Différentes mesures de l'activité du système nerveux autonome peuvent opérer indépendamment ou en opposition l'une à l'autre. La captation des émotions par ce moyen s'opère grâce à l'étude de signaux physiologiques tels que l'activité électro-dermale (EDA) et le rythme cardiaque (BR). Pour autant, cette méthode nécessite un grand nombre de mesures de sources différentes et complexifie ainsi les dispositifs.

La troisième méthode consiste à calculer la magnitude de réponse à un stimulus inattendu pour mesurer la valence dans un contexte spécifique avec un stimulus de haute intensité. La magnitude est sensible à la valence uniquement dans le contexte d'un stimulus de haute excitation. Ainsi, cette méthode n'est pas adaptée pour la détection d'un état émotionnel discret ou de moins haute excitation.

La quatrième méthode repose sur l'étude du système nerveux central (SNC) à l'aide d'électroencéphalogramme (EEG) et d'imagerie cérébrale. Cette méthode nécessite des infrastructures lourdes et n'est pas adaptée au quotidien. Les coûts de mise en œuvre sont importants, et les expériences ont un caractère très invasif qui peut dissuader les individus d'y participer. Ces éléments rendent alors ces techniques difficiles à contrôler et à valider.

La dernière méthode se base sur le comportement corporel qui traduit la réponse du corps à l'émotion (ex. analyse de la voix ou expression du visage). Néanmoins, cette dernière méthode est complexe à mettre en œuvre pour un usage quotidien. Dès lors, il existe un besoin pour permettre une meilleure détection des émotions, à l'aide d'un nombre restreint de mesures (ex. une seule source de mesures), tout en permettant de détecter de manière fiable la valence des émotions.

<u>État de la technique</u>

On connaît dans l'état de la technique la demande de brevet coréenne KR20160085577 décrivant un appareil pour déterminer un état psychologique comprenant: une unité d'acquisition de bio-signal pour acquérir un premier biosignal et un second bio-signal d'un utilisateur généré par un premier stimulus; et une unité de détermination d'état psychologique pour extraire une première caractéristique et une deuxième caractéristique en analysant le premier biosignal et le deuxième bio-signal et en déterminant l'état psychologique de l'utilisateur sur la base de la première caractéristique et de la deuxième caractéristique. Le premier bio-signal et le second bio-signal sont des bio-signaux indiquant des changements dans les nerfs autonomes mesurés par différents bio-capteurs.

On connaît aussi la demande de brevet américaine US2008214903 décrivant une solution mettant en œuvre un ou plusieurs modules capteurs portables pour transmettre le ou les paramètres physiologiques. Un ou plusieurs émetteurs transmettent sans fil à un moniteur mobile des signaux signalés les valeurs desdits un ou plusieurs paramètres physiologiques. Le moniteur mobile comprend un processeur traitant les signaux reçus de l'émetteur en temps réel en utilisant les avoir de l'expert. Un dispositif fournit une ou plusieurs indications sur les résultats du traitement. Cette demande concerne également des capteurs mobiles portables à utiliser dans le système décrit. Le procédé consiste à obtenir les valeurs des paramètres physiologiques de l'utilisateur auprès d'un ou plusieurs modules capteurs portables.

Les signaux signalés les valeurs du ou des paramètres physiologiques sont transmis sans fil à un moniteur mobile. Les signaux sont traités en temps réel au moyen du savoir de l'expert et une ou plusieurs indications de résultats du traitement sont fournies à l'unité mobile.

Le brevet US2019239795 décrit un procédé comprenant les étapes suivantes: le stockage d'informations sur une émotion du sujet, et d'informations sur une activité du sujet; la génération de données d'apprentissage représentant une relation entre les informations stockées émotion du sujet, et les informations stockées l'activité du sujet et le stockage des données d'apprentissage en mémoire, après que les données d'apprentissage sont générées, d'une émotion actuelle du sujet sur la base des informations relatives à une activité actuelle du sujet obtenu par une unité d'obtention, et les données d'apprentissage stockées en mémoire; et la fourniture d'une assistance à la conduite du véhicule sur la base de l'émotion actuelle. De la même manière, ce document des appareils de commande de chaîne de fabrication et d'aide aux soins de santé, les appareils permettant de fournir une commande de chaîne de fabrication et une aide aux soins de santé sur la base de l'émotion estimée. L'appareil estime les variations émotionnelles du sujet à l'aide desdites équations de régression, et des variations des caractéristiques caractéristiques des éléments de données de mesure, ou de l'activité électrique cardiaque (H), de l'activité du potentiel cutané (G), du mouvement oculaire (EM), du mouvement (BM) et de la quantité d'activité (Ex) du sujet mesuré par le dispositif de mesure.

La demande de brevet US2016345060 décrit la mesure, via des capteurs, des réponses d'un individu au contenu pendant une première fois, la détermination de classifications de réponse sur la base d'une comparaison des réponses et des seuils respectifs, la détermination d'une première classification mentale de l'individu basée sur la combinaison des classifications de réponse, la détermination une ligne de base pendant la première fois, mesurer des réponses supplémentaires au contenu pendant une seconde fois, déterminer des classifications de réponses supplémentaires sur la base d'une comparaison des réponses supplémentaires à des seuils supplémentaires respectifs, ajuster la ligne de base sur la base des réponses supplémentaires dans la seconde fois.

On connaît aussi les articles suivants :

- A machine learning model for emotion récognition from physiological signals Author J.A.Domínguez-JiménezK.C.Campo-LandinesJ.C.Martinez-SantosE.J.DelahozS.H.Contreras-OrtizUniversidad Tecnológica de Bolívar, Km 1 Vía. Turbaco, Cartagena de Indias, Colombia Received 6 December 2018, Revised 26 June 2019, Accepted 7 August 2019, Available online 3 September 2019.

- A data driven empirical itérative algorithm for GSR signal pre-processing auteurs Arvind Gautam, Neide Simoes-Capela, Giuseppina Schiavorie, Amit Acharyya, Walter De Raedt, Chris Van Hoof publié 2018/9/3 dans 2018 26th European Signal Processing Conférence (EUSIPCO)Pages 1162-1166

Inconvénients de l'art antérieur

Les solutions de l'art antérieur ne permettent pas de fournir un signal réellement représentatif de l'état émotionnels en prenant en compte les facteurs sous-jacents comme la valence et le niveau d'éveil («arousal»), qui sont pourtant déterminants pour une caractérisation pertinente de l'état émotionnel, d'une manière automatique et sans intervention d'un humain formé à l'évaluation psychologique de l'état émotionnel ni de fournir une information fiable, robuste et reproductible.

Aujourd'hui, il n'existe pas de système sous forme de bracelet connecté qui permette d'identifier en temps réel l'état émotionnel à partir de données physiologiques (activité cardiaque notée PPG pour photoplethysmography, conductance électrodermale notée GSR pour galvanic skin response. En effet, les dispositifs/systèmes existants utilisent des algorithmes de classification (apprentissage automatique) qui nécessitent une analyse fréquentielle de l'activité cardiaque sur une durée de l'ordre de 5 minutes.

De plus, la performance des algorithmes de classification actuels est fortement limitée par les protocoles d'induction émotionnelle trop spécifiques d'une part et par les méthodes de labélisation émotionnelle des données physiologiques à partir de mesures subjectives (questionnaires, échelles de likert, etc.) d'autre part. En effet, les variations inter-individuel dans la représentation subjective des émotions couplées aux variations inter-individuel dans la qualité des signaux physiologique ne permettent pas de classifier de façon robuste et non contextualisée les émotions.

Enfin, l'expression physiologique des émotions ne doit pas être évaluée de façon trop spécifique comme la plupart des laboratoires de recherche tentent de le faire car un groupe d'émotions peut produire des réponses physiologiques similaires. Par exemple, la réaction de surprise ou l'attirance sexuelle produisent des signaux GSR ayant des caractéristiques similaires. Il est préférable d'appréhender l'expression physiologique des émotions en tenant compte du réel fonctionnement du système nerveux autonome qui joue un rôle fondamental dans l'adaptation aux émotions. Les deux branches activatrices et inhibitrices du système nerveux autonome (respectivement les systèmes nerveux sympathiques et parasympathiques) agissent de façon antagoniste comme une balance dynamique. Cette balance permet d'appréhender les différents groupes émotionnels et peut être caractérisée à partir de mesures non invasives.

Solution apportée par l'invention

Afin de remédier à ces inconvénients, l'invention concerne selon son acception la plus générale un procédé mis en œuvre par ordinateur de calcul d'un couple de données numériques représentatives d'un état émotionnel consistant :

- à acquérir :

  ◦ une première série de signaux physiologiques par au moins un capteur d'activité électrodermal EDA

  ◦ une deuxième série de signaux physiologiques par photopléthysmographie (PPG) ; La photopléthysmographie est une technique d'exploration fonctionnelle vasculaire non invasive utilisée notamment par les montres et bracelet connectés pour mesurer la fréquence cardiaque du porteur de la montre.

- à transmettre à un serveur distant lesdits signaux horodatés ainsi qu'un identifiant de l'équipement d'acquisition

- à procéder à un traitement de chacun desdits signaux pour caractériser un couple de données caractérisé en ce que

- ledit traitement de ladite première série de signaux est de type EMD (décomposition modale empirique) sur une fenêtre temporelle glissante dont le résultat fourni la première valeur dudit couple (arousal)

- ledit traitement de ladite deuxième série de signaux comporte une étape de filtrage passe-bande des fréquences comprises entre 0,04 et 0,26 Hz et de détection de pics et de mesure temporelle inter-pic RR, sur ladite fenêtre temporelle glissante dont le résultat fourni la deuxième valeur dudit couple (valence).

De préférence, lesdites séries de données GSR et PPG sont horodatées et transmises sous forme de messages numériques à un calculateur effectuant les calculs pour le calcul des valeurs $S_{arousal}$, $S_{valence}$ en temps réel.

Avantageusement, lesdites séries de données GSR et PPG sont stockées dans des buffers de calcul dont les mémoires tampons pour l'application des traitements de fenêtres temporelles glissantes.

Selon une variante, le procédé comporte une étape de filtrage passe-bande (butterworth de 4ème ordre) dudit signal signal GSR avec une bande passante comprise entre 0.05 et 1Hz.

Avantageusement, le traitement pour la détermination dudit signal $S_{Arousal}$ est effectué sur une fenêtre temporelle de 15 à 25 secondes s à partir de la puissance spectrale normalisée du signal GSR calculée sur la bande 0.045 - 0.25Hz.

Selon une autre variante, le procédé comporte une étape de filtrage passe-bande (butterworth de 4ème ordre) dudit signal PPG avec une bande passante comprise entre 0.5 et 5Hz.

Selon un mode de mise en œuvre particulier, le procédé comporte en outre une étape de visualisation consistant à commander l'affichage d'une forme graphique dont un premier paramètre est fonction de la valeur dudit signal $S_{arousal}$ et dont un second paramètre est fonction de la valeur dudit signal $S_{valence}$.

Avantageusement, ledit premier paramètre comprend la taille, l'épaisseur du contour ou le facteur de forme, et en ce que ledit second paramètre comprend la couleur et l'orientation d'un axe principal de ladite forme graphique.

Selon un autre mode de mise en oeuvre avantageux, le procédé comporte une étape préalable d'apprentissage supervisé consistant à présenter à un panel de personnes équipées d'un dispositif d'acquisition desdits signaux physiologiques GSR et/ou EDA et PPG une pluralité de plans d'expériences formés par une succession de séquences vidéos associées chacune à un descripteur numérique ID (t), et d'enregistrer les couples de signaux $S_{arousal}$ et $S_{valence}$ et leur évolution dans le temps, pour chacun des membres du panel, puis à) injecter les données structurées ($S_{arousal}$ et $S_{valence}$ (t) ; ID (t)) dans un réseau de neurones, pour élaborer un modèle de caractérisation.

Description détaillée d'un exemple non limitatif de l'invention

La présente invention sera décrite dans ce qui suit à titre d'exemple non limitatif, illustré par les dessins annexés où :

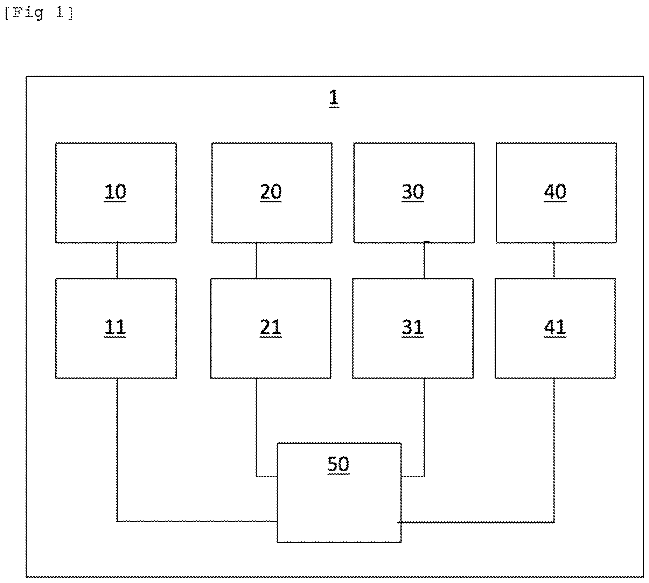

[Fig 1] la figure 1 représente un exemple de dispositif d'acquisition des signaux électrophysiologiques.

Contexte de l'information

Le but de l'invention est de fournir automatiquement et sans intervention d'un humain un couple de signaux numériques $S_{arousal}$ et $S_{valence}$ représentatifs de l'état émotionnel d'une personne.

La reconnaissance efficiente des émotions à partir de l'activité physiologique humaine peut passer par l'utilisation d'un modèle émotionnel simple. En effet, les émotions peuvent être projetées dans un espace multidimensionnel, le plus commun étant le plan valence-éveil. Le niveau de valence représente la positivité et la négativité d'une émotion alors que le niveau d'éveil (arousal en anglais) décrit l'intensité de l'émotion. Ces deux composantes émotionnelles sont exprimées au niveau physiologique.

Lors d'un stress, le système nerveux sympathique prédomine et conduit à une élévation du niveau d'éveil physiologique. Une accélération de la fréquence cardiaque ou une accélération de l'intervalle inter-battement (IBI Interbeat interval) est caractéristique de cet état. Au repos, au contraire, le système nerveux parasympathique s'active traduisant une diminution de l'état d'éveil physiologique et de la fréquence cardiaque. Par ailleurs, l'alternance des accélérations et des décélérations de la fréquence cardiaque devient régulière et cohérente (état de cohérence cardiaque) dans les états de bien-être, de calme, de maîtrise (valence émotionnelle positive) alors que dans les états de stress, d'anxiété, de colère (valence émotionnelle négative), le tachogramme correspondant au couple $S_{arousal}$, et $S_{valence}$ devient irrégulier, son tracé chaotique et sa magnitude va diminuer.

En extrayant du signal PPG le niveau de cohérence du rythme cardiaque, il devient possible d'obtenir un indicateur robuste du niveau de valence émotionnelle et de calculer des seuils dynamiques au-delà desquels ce niveau valence change de façon significative.

Une fois le niveau de valence estimé, il est alors possible de vérifier le niveau d'éveil en contrôlant dans le domaine spectral le niveau d'activation physiologique à partir du signal GSR pour en déduire en temps réel l'état émotionnel de l'individu et le communiquer au système multimédia avec lequel ce dernier interagit.

On peut caractériser l'état émotionnel selon le tableau suivant :

[Table 1]

| | $S_{valence}$ | | | | + | |
|---|---|---|---|---|---|---|
| + | | | CRISPE | ALERTE | | |
| $S_{arousal}$ | | NERVEUX | | | EXITE | |
| | CONTRARIE | | | | | |
| | TRISTE | | | | | HEUREUX |
| | DREPRESSIF | | | | CONTENT | |
| - | | ENNUYE | | SEREIN | | |
| | | | RELAXE | | | |

Lors d'un stress, le système nerveux sympathique prédomine et conduit à une élévation du niveau d'éveil physiologique. Une accélération de la fréquence cardiaque est caractéristique de cet état. Au repos, au contraire, le système nerveux parasympathique s'active traduisant une diminution de l'état d'éveil physiologique et de la fréquence cardiaque. Par ailleurs, l'alternance des accélérations et des décélérations de la fréquence cardiaque devient régulière et cohérente (état de cohérence cardiaque) dans les états de bien-être, de calme, de maîtrise (valence émotionnelle positive) alors que dans les états de stress, d'anxiété, de colère (valence émotionnelle négative), le tachogramme devient irrégulier, son tracé chaotique et sa magnitude va diminuer.

En extrayant du signal PPG le niveau de cohérence du rythme cardiaque, il devient possible d'obtenir un indicateur robuste du niveau de valence émotionnelle et de calculer des seuils dynamiques au-delà desquels ce niveau valence change de façon significative.

Une fois le niveau de valence estimé, il est alors possible de vérifier le niveau d'éveil en contrôlant dans le domaine spectral le niveau d'activation physiologique à partir du signal GSR [4] pour en déduire en temps réel l'état émotionnel de l'individu et le communiquer au système multimédia avec lequel ce dernier interagit.

Dispositif d'acquisition des signaux électrophysiologiques

Le dispositif d'acquisition présente une surface de contact cutanée comportant les capteurs. Elle peut être prévue à la surface d'un support tel qu'un bracelet destiné à être porté au bras ou à la cheville, le dos d'une montre, ou encore de patch pouvant être fixé sur la peau de l'utilisateur.

La surface de contact cutanée (1) du dispositif d'acquisition comporte une pluralité de capteurs (10, 20, 30, 40) destinés l'obtention de mesures de signaux physiologiques associés aux émotions de l'utilisateur, par exemple :

- un capteur (10) apte à la mesure d'un rythme cardiaque de l'utilisateur.

- un capteur (20) apte à mesurer de la conductivité électrique observée à la surface de la peau de l'utilisateur et fournissant un signal représentatif de l'activité électrodermale (EDA)

- un capteur (30) apte à la mesure de la température superficielle de la peau

- un capteur (40) constitué par un accéléromètre trois-axes ou multi-axes tels qu'un module inertiel 9 axes apte à permettre la mesure de mouvements sur un membre de l'utilisateur.

Le capteur (20) fournit un signal représentatif d'un paramètre passif ou endosomatique correspondant au niveau de conductance dermique (skin conductance level SCL) ou d'un paramètre actif ou exoosomatique correspondant au niveau de la réponse de conductance dermique (skin conductance responses SCR). Ces paramètres permettent de déterminer l'activité électrodermale (AED) trouvant son origine des caractéristiques de la membrane épidermique, et de l'activité sudoripare de type eccrine sous contrôle des systèmes nerveux autonome et central.

On distingue deux méthodes d'enregistrement.

La première méthode dite endosomatique traduit les différences de potentiels générées par les membranes cutanés et conduit à la mesure du potentiel électrodermal. Dans ce cas, le capteur (20) est un capteur de conductivité de la peau associé à un convertisseur courant-tension, par exemple un capteur de résistivité de la peau, muni d'une paire d'électrodes en acier noble.

La seconde méthode dite exosomatique traduit les variations d'un courant appliqué à la peau dont les caractéristiques peuvent conduire à la mesure de divers signaux électrodermaux dont la mesure de conductance cutanée, la plus couramment utilisée dans la littérature. Chacun des signaux électrodermaux se subdivise en une composante tonique et une composante phasique.

La première identifie les variations lentes du signal électrodermal alors que la seconde correspond aux variations rapides du signal communément appelées réponses électrodermales. De ces mesures phasiques peuvent être extraits différents paramètres de mesure tels que la fréquence, la latence ou l'amplitude des réponses électrodermales. Les origines ainsi que la variabilité des paramètres de mesure de l'activité électrodermale font de cette activité une mesure sensible aux changements de notre environnement et à différents processus mentaux sous contrôle du système nerveux central tels que l'émotion, la motivation ou encore l'attention, et la charge mentale.

Chaque capteur (10, 20, 30, 40) est associé à un circuit de prétraitement (11, 21, 31, 41) réalisant optionnellement un traitement analogique (préemplification, filtration, délivrance d'un signal d'excitation,), de numérisation (échantillonnage, filtrage numérique optionnel, stockage dans une mémoire tampon,...) pour délivrer à un calculateur (50) des signaux numériques exploités pour déterminer le couple de valeurs représentatives de l'état émotionnel.

Traitements des signaux physiologiques

Les signaux fournis par le capteur (40) sont échantillonnés à une fréquence de 64 Hz et filtrés en amplitude et en fréquence pour supprimer les signaux aberrants. Ces signaux constituent des informations environnementales venant compléter les signaux associés aux émotions, par exemple pour donner un contexte de mode de déplacement et/ou de chute.

Les signaux fournis par le capteur (20) de conductivité électrique est échantillonné à une fréquence de 8 Hz et ensuite traité pour le calcul du score d'arousal et le niveau de vigilance.

Les signaux fournis par le capteur (10) de rythme cardiaque de l'utilisateur sont échantillonnés à une fréquence de 50 Hz et exploités par le calculateur (50) pour la détermination du score de valence, ainsi que pour la reconnaissance biométrique et l'estimation du niveau de stress.

Le capteur (30) de mesure de la température de la peau est échantillonné à une fréquence basse de l'ordre de 1Hz et complète les informations permettant de caractériser l'état émotionnel.

Exemple de mise en œuvre particulier

Un exemple de mise en œuvre consiste à équiper le patient d'un bracelet connecté sans fil équipé de trois capteurs physiologiques (un seul capteur peut suffire) mesurant la conductance électrodermale (notée GSR pour galvanic skin response) à une cadence 8Hz, l'activité cardiaque (notée PPG pour photoplethysmography) à une cadence 50Hz, la température corporelle (notée SKT pour skin température) à une cadence 1Hz et d'un ou plusieurs capteurs accélérométriques (notés ACC) à une cadence de 50Hz est utilisé pour enregistrer de façon synchrone les données et les time stamps correspondants.

Les données GSR et PPG sont transmises à un terminal mobile qui effectue les calculs pour l'identification en temps réel de l'état émotionnel. Les données GSR et PPG sont stockées dans des tampons de calcul dont les durées varient selon les variables calculées.

Dans chaque mémoire tampon, le traitement du signal est effectué avant l'extraction des différentes variables utilisées pour l'analyse d'identification de l'état émotionnel :

Signal GSR : Un filtrage passe-bande (butterworth de 4ème ordre) est appliqué au signal avec une bande passante de 0.05 - 1Hz.

Signal PPG : Un filtrage passe-bande (butterworth de 4ème ordre) est appliqué au signal avec une bande passante de 0.5 - 5Hz.

Les variables utilisées pour l'analyse d'identification de l'état émotionnel sont ensuite extraites des signaux traités. La variable *Arousal* est obtenue dans une mémoire tampon de calcul de 20 secondes à partir de la puissance spectrale normalisée du signal GSR calculée sur la bande 0.045 - 0.25Hz par une transformation Hilbert-Huang.

Exemple de traitement

La variable *Mdiff* est enregistrée dans un tampon de calcul de 2 secondes à partir de moyenne de la valeur absolue de la dérivée première du signal GSR.

La variable *Valence* est obtenue dans un tampon de calcul de 60 secondes en calculant le ratio de cohérence cardiaque. Pour cela, une détection des pics dans le signal PPG est réalisée à partir d'une fonction dédiée pour en déduire les intervalles de temps pic à pic notés intervalles RR. Puis, le rythme cardiaque noté *BPM* est calculé à partir des intervalles RR.

A partir du signal *BPM*, le pic maximum du spectre de puissance est identifié sur la bande 0.04 - 0.26Hz (la gamme de fréquence au sein de laquelle la cohérence peut être observée). La puissance de ce pic notée *Peak Power* est ensuite déterminé en calculant l'intégrale sur une fenêtre de 0.030Hz de large, centrée autour du pic. La puissance totale sur la bande 0.0033 - 0.4Hz du signal BPM notée *Total Power* est ensuite calculée. Le niveau de valence normalisé est obtenu par le calcul suivant :

[Math 1]

$$Valence = \frac{Peak\ Power}{Total\ Power - Peak\ Power} \qquad (1)$$

Toutes les secondes, les nouvelles valeurs GSR et PPG enregistrées par le bracelet permettent de calculer les nouvelles valeurs *Arousal, Mdiff* et *Valence*.

Mdiff est stockée en mémoire la dernière minute pour permettre une calibration dynamique du système de détection des variations ponctuelles du niveau d'excitation physiologique. Un coefficient de pondération est appliqué à ces données de calibration pour rendre la contribution des valeurs les plus récentes plus importante lors de la calibration. Il est ensuite possible de calculer les seuils dynamiques permettant de classifier respectivement la variable *Mdiff.* Le calcul des seuils peut être détaillé de la façon suivante :

[Math 2]

$$Seuil_{(t)} = \frac{Max\left(Mdiff\begin{bmatrix} x_1 \\ \vdots \\ x_n \end{bmatrix}\right) + Moyenne\left(Mdiff\begin{bmatrix} x_1 \\ \vdots \\ x_n \end{bmatrix}\right)}{2} \qquad (1)$$

Avec *Seuil*$_{(t)}$ la valeur du seuil dynamique à l'instant *t* et *Mdiff* $\begin{bmatrix} x_1 \\ \vdots \\ x_n \end{bmatrix}$ les valeurs de la variable *Mdiff* sur toute la durée de la période de calibration.

Chaque seconde, une nouvelle valeur de *Seuil* est obtenue et comparée à *Mdiff*. Si *Mdiff* est supérieure à sa valeur seuil, alors une réaction émotionnelle est détectée.

Apprentissage des critères de caractérisation

Pour construire un modèle de caractérisation, l'invention propose une variante mettant en œuvre une étape préparatoire d'apprentissage supervisé.

Cette solution consiste à proposer à un panel d'usagers équipé d'un dispositif d'acquisition des données physiologiques susvisé, des plans d'expériences formés par une succession de séquences vidéos associées chacune à un descripteur numérique ID (t), et d'enregistrer les couples de signaux $S_{arousal}$ et $S_{valence}$ et leur évolution dans le temps, pour chacun des membres du panel.

Ces données structurées ($S_{arousal}$ et $S_{valence}$ (t) ; ID (t)) pour chacun des membres du panel sont ensuite injectés dans un réseau de neurones, pour élaborer un modèle de caractérisation.

Les participants seront équipés d'un bracelet connecté conforme à l'invention équipés de trois capteurs physiologiques mesurant l'activité cardiaque (notée PPG pour photoplethysmography), la température corporelle (notée SKT pour skin température) et conductance électrodermale (notée GSR). Les bracelets communiquent avec un ordinateur portable d'acquisition permettant d'enregistrer de façon synchrone les données et l'horodatage correspondant avec une fréquence d'acquisition de 50Hz, 1Hz et 4Hz pour le PPG, la SKT et le GSR, respectivement pour le bracelet connecté ou montre connectée et avec une fréquence d'acquisition de 64Hz, 4Hz et 4Hz pour le PPG, la SKT et le GSR.

Un système de réalité virtuelle HTC Vive sera utilisé pour afficher les stimuli sélectionnés pour induire une réaction émotionnelle et permet d'avoir une immersion supplémentaire (inédit en protocole de stimulation émotionnel)

Design expérimental

Pour chaque participant, les données seront enregistrées sur une seule session de vingt minutes. Le plan d'expérience est : Sn (participants) * V6 (six vidéos émotionnelles).

Chaque vidéo correspond à un extrême émotionnel:

- Vidéo 1: Repos (40s) - Phase d'induction émotionnelle de type tristesse (30s) - Post-effet (30s)

- Vidéo 2; Repos (40s) - Phase d'induction émotionnelle de type joie (30s) - Post-effet (30s)

- Vidéo 3; Repos (40s) - Phase d'induction émotionnelle de type dégoût (30s) - Post-effet (30s)

- Vidéo 4; Repos (40s) - Phase d'induction émotionnelle de type peur (30s) - Post-effet (30s)

- Vidéo 5; Repos (40s) - Phase d'induction émotionnelle de type neutre (30s) - Post-effet (30s)

- Vidéo 6: Repos (40s) - Phase d'induction émotionnelle de type relaxation (30s) - Post-effet (30s)

La phase repos constituera une période de référence pour initialiser le calcul des variables physiologiques. Pour chaque participant, l'ordre de présentation des vidéos sera aléatoire afin d'éviter tout effet d'ordre. De plus, afin d'enrichir le jeu de données, deux vidéos seront disponibles pour les émotions de type peur et joie. Pour chaque participant, le choix de la vidéo utilisée pour chacune de ces deux émotions sera aléatoire.

Procédure d'acquisition des données

Chaque participant est d'abord équipé d'un ou plusieurs bracelets connectés et d'un système de réalité virtuelle lui permettant de s'isoler des stimulations extérieures et d'optimiser son focus attentionnel. L'expérimentateur vérifie ensuite la qualité des signaux physiologiques. Chaque participant aura pour instruction générale de visualiser six vidéos d'une durée de 30 secondes. Lors des 40 secondes précédent la vidéo et des 30 secondes suivant la vidéo, la consigne donnée sera de rester calme et immobile. Lorsque toutes les vidéos ont été visualisées, l'expérimentateur aide le participant à enlever le casque de réalité virtuelle et le bracelet puis réalise un débriefing pour vérifier que tout s'est bien passé.

Analyse des données

Pour chaque participant, les données physiologiques enregistrées seront prétraitées de la façon suivante : Pour le signal PPG, les sauts de signal seront corrigés à l'aide d'une fonction dédiée. Un filtrage passe-bande (butterworth de 4ème ordre) sera ensuite appliqué au signal avec une bande passante de 0.5 - 5Hz puis le signal sera normalisé à l'aide d'une transformée d'Hilbert et lissé à l'aide d'une fenêtre gaussienne de 16 secondes. En ce qui concerne le signal SKT, un filtrage passe-bas (butterworth de 4ème ordre) sera appliqué au signal avec une fréquence de coupure à 0.05Hz. Enfin, un filtrage passe-bande (butterworth de 4ème ordre) sera appliqué au signal GSR avec une bande passante de 0.05 - 3Hz. Toutes ces variables constitueront les données d'entrée des algorithmes de classification émotionnelle.

Représentation graphique des résultats des traitements

Toutes les secondes, les variables obtenues sont représentées par le système d'affichage de l'état émotionnel détecté (noté overlay) de la façon suivante :

Le diamètre du cercle correspond à la valeur normalisée de la valeur $S_{Arousal}$. Plus le diamètre est important, plus le niveau d'éveil est élevé.

La couleur du cercle correspond à la valeur normalisée de la valeur $S_{valence}$. Lorsque la couleur tend vers le vert, le niveau de valence est plus élevé. Lorsque la couleur tend vers le rouge, le niveau de valence est plus faible.

Lorsqu'une une réaction émotionnelle est détectée à partir du paramètre d'évolution *Mdiff,* le contour du cercle s'anime. La valeur du rythme cardiaque s'actualise au centre du cercle.

L'état émotionnel est communiqué au système multimédia avec lequel l'individu interagit. Il est important de noter que le dock/mobile prévoit la mise à jour du bracelet d'une part et des méthodes de calculs des valeurs et des niveaux de détection d'autre part via une connexion internet.

Applications

Le procédé selon l'invention permet de délivrer des signaux de commande pour le pilotage d'un équipement tel qu'un robot, notamment un robot empathique ou de commande de paramètres fonctionnels d'un équipement électronique tel que le niveau sonore, lumineux, le rythme...

Ces signaux permettent également de commander l'adaptation de la vitesse d'un véhicule de transport individuel ; collectif et la gestion des agents de sécurité, de contrôle, pilotes, conducteurs.

**Revendications**

**1.** - Procédé de calcul d'un couple de données numériques représentatives d'un état émotionnel consistant ; à acquérir :

- une première série de signaux physiologiques par au moins un capteur électro-dermique GSR et/ou EDA
- une deuxième série de signaux physiologiques PPG par un capteur de rythme cardiaque
- à transmettre à un serveur distant lesdits signaux horodatés ainsi qu'un identifiant de l'équipement d'acquisition
- à procéder à un traitement de chacun desdits signaux pour caractériser un couple de données $S_{arousal}$, $S_{valence}$
- ledit traitement de ladite première série de signaux étant de type décomposition modale empirique (EMD) sur une fenêtre temporelle glissante dont le résultat fourni la première valeur $S_{arousal}$ dudit couple
- ledit traitement de ladite deuxième série de signaux comportant une étape de filtrage passe-bande des fréquences comprises entre 0,04 et 0,26 Hz et de détection de pics et de mesure temporelle inter-pic RR, sur ladite fenêtre temporelle glissante dont le résultat fourni la deuxième valeur $S_{valence}$ dudit couple.

**2.** - Procédé de calcul d'un couple de données numériques représentatives d'un état émotionnel selon la revendication 1 **caractérisé en ce que** lesdites séries de données GSR et PPG sont horodatées et transmises sous forme de messages numériques à un calculateur effectuant les calculs pour le calcul des valeurs $S_{arousal}$, $S_{valence}$ en temps réel.

**3.** - Procédé de calcul d'un couple de données numériques représentatives d'un état émotionnel selon la revendication 1 **caractérisé en ce que** lesdites séries de données GSR et PPG sont stockées dans des buffers de calcul dont les mémoires tampons pour l'application des traitements de fenêtres temporelles glissantes.

**4.** - Procédé de calcul d'un couple de données numériques représentatives d'un état émotionnel selon la revendication 1 **caractérisé en ce qu'**il comporte une étape de filtrage passe-bande dudit signal signal GSR avec une bande passante comprise entre 0.05 et 1Hz.

**5.** - Procédé de calcul d'un couple de données numériques représentatives d'un état émotionnel selon la revendication précédente **caractérisé en ce que** le traitement pour la détermination dudit signal $S_{Arousal}$ est effectué sur une fenêtre temporelle de 15 à 25 secondes s à partir de la puissance spectrale normalisée du signal GSR calculée sur la bande 0.045 - 0.25Hz.

6. - Procédé de calcul d'un couple de données numériques représentatives d'un état émotionnel selon la revendication 1 **caractérisé en ce qu'**il comporte une étape de filtrage passe-bande dudit signal PPG avec une bande passante comprise entre 0.5 et 5Hz.

7. - Procédé de calcul d'un couple de données numériques représentatives d'un état émotionnel selon la revendication 1 **caractérisé en ce qu'**il comporte une étape de visualisation consistant à commander l'affichage d'une forme graphique dont un premier paramètre est fonction de la valeur dudit signal $S_{arousal}$ et dont un second paramètre est fonction de la valeur dudit signal $S_{valence}$.

8. - Procédé de calcul d'un couple de données numériques représentatives d'un état émotionnel selon la revendication précédente **caractérisé en ce que** ledit premier paramètre comprend la taille, l'épaisseur du contour ou le facteur de forme, et **en ce que** ledit second paramètre comprend la couleur et l'orientation d'un axe principal de ladite forme graphique.

9. - Procédé de calcul d'un couple de données numériques représentatives d'un état émotionnel selon la revendication 1 **caractérisé en ce qu'**il comporte une étape préalable d'apprentissage supervisé consistant à présenter à un panel de personnes équipées d'un dispositif d'acquisition desdits signaux physiologiques GSR et/ou EDA et PPG une pluralité de plans d'expériences formés par une succession de séquences vidéos associées chacune à un descripteur numérique ID (t), et d'enregistrer les couples de signaux $S_{arousal}$ et $S_{valence}$ et leur évolution dans le temps, pour chacun des membres du panel, puis à) injecter les données structurées ($S_{arousal}$ et $S_{valence}$ (t) ; ID (t)) dans un réseau de neurones, pour élaborer un modèle de caractérisation.

**Patentansprüche**

1. Verfahren zum Berechnen eines Paars digitaler Daten, die für einen emotionalen Zustand repräsentativ sind, bestehend aus:
   Erfassen von:

   - einer ersten Reihe von physiologischen Signalen durch mindestens einen elektrodermalen GSR- und/oder EDA-Sensor
   - einer zweiten Reihe von physiologischen PPG-Signalen durch einen Herzrhythmussensor
   - Übertragen der mit Zeitstempel versehenen Signale sowie einer Kennung der Erfassungsausrüstung an einen entfernten Server
   - Durchführen einer Verarbeitung jedes dieser Signale zum Kennzeichnen eines Paars digitaler Daten $S_{arousal}$, $S_{valence}$
   - wobei das Verarbeiten der ersten Reihe von Signalen ein Typ der empirischen Modenzerlegung (EMD) über ein gleitendes Zeitfenster ist, deren Ergebnis den ersten Wert $S_{arousal}$ des Paars liefert
   - wobei das Verarbeiten der zweiten Reihe von Signalen einen Schritt eines Bandpassfilterns von Frequenzen zwischen 0,04 und 0,26 Hz und eines Erkennens von Peaks und eines zeitlichen Messens zwischen Peaks RR über das gleitende Zeitfenster, dessen Ergebnis den zweiten Wert $S_{valence}$ des Paars liefert.

2. Verfahren zum Berechnen eines Paars digitaler Daten, die für einen emotionalen Zustand repräsentativ sind, nach Anspruch 1,
   **dadurch gekennzeichnet, dass** die Reihen von GSR- und PPG-Daten mit einem Zeitstempel versehen und in Form von digitalen Nachrichten an einen Rechner, der die Berechnungen für die Berechnung der Werte $S_{arousal}$, $S_{valence}$ in Echtzeit ausführt, übertragen werden.

3. Verfahren zum Berechnen eines Paars digitaler Daten, die für einen emotionalen Zustand repräsentativ sind, nach Anspruch 1,
   **dadurch gekennzeichnet, dass** die Reihen von GSR- und PPG-Daten in Berechnungspuffern gespeichert werden, von denen die Pufferspeicher der Anwendung der Verarbeitungen der gleitenden Zeitfenster dienen.

4. Verfahren zum Berechnen eines Paars digitaler Daten, die für einen emotionalen Zustand repräsentativ sind, nach Anspruch 1,
   **dadurch gekennzeichnet, dass** es einen Schritt des Bandpassfilterns des Signals GSR-Signals mit einer Bandbreite zwischen 0,05 und 1 Hz aufweist.

5. Verfahren zum Berechnen eines Paars digitaler Daten, die für einen emotionalen Zustand repräsentativ sind, nach dem vorstehenden Anspruch, **dadurch gekennzeichnet, dass** das Verarbeiten für die Bestimmung des Signals $S_{Arousal}$ in einem Zeitfenster von 15 bis 25 Sekunden s von der normalisierten Spektralleistung des GSR-Signals, die in dem Band 0,045 - 0,25 Hz berechnet wird, ausgeführt wird.

6. Verfahren zum Berechnen eines Paars digitaler Daten, die für einen emotionalen Zustand repräsentativ sind, nach Anspruch 1,
   **dadurch gekennzeichnet, dass** es einen Schritt des Bandpassfilterns des PPG-Signals mit einer Bandbreite zwischen 0,5 und 5 Hz aufweist.

7. Verfahren zum Berechnen eines Paars digitaler Daten, die für einen emotionalen Zustand repräsentativ sind, nach Anspruch 1,
   **dadurch gekennzeichnet, dass** es einen Visualisierungsschritt, der darin besteht, die Anzeige einer grafischen Form zu steuern, von der ein erster Parameter von dem Wert des Signals $S_{arousal}$ abhängig ist und von dem ein zweiter Parameter von dem Wert des Signals $S_{valence}$ abhängig ist, aufweist.

8. Verfahren zum Berechnen eines Paars digitaler Daten, die für einen emotionalen Zustand repräsentativ sind, nach dem vorstehenden Anspruch, **dadurch gekennzeichnet, dass** der erste Parameter die Größe, die Dicke der Kontur oder den Formfaktor umfasst, und dass der zweite Parameter die Farbe und die Ausrichtung einer Hauptachse der grafischen Form umfasst.

9. Verfahren zum Berechnen eines Paars digitaler Daten, die für einen emotionalen Zustand repräsentativ sind, nach Anspruch 1,
   **dadurch gekennzeichnet, dass** es einen vorherigen Schritt eines überwachten Lernens, der darin besteht, einem Panel von Personen, die mit einer Vorrichtung zum Erfassen der physiologischen GSR- und/oder EDA- und PPG-Signale ausgestattet sind, eine Vielzahl von Versuchsplänen zu präsentieren, die durch eine Folge von Videosequenzen ausgebildet werden, die jeweils mit einem digitalen Deskriptor ID (t) verknüpft sind, und des Speicherns der Signalpaare $S_{arousal}$ und $S_{valence}$ und ihrer zeitlichen Entwicklung für jedes der Panelmitglieder, aufweist, wobei dann) die strukturierten Daten ($S_{arousal}$ und $S_{valence}$ (t); ID (t)) in ein neuronales Netz eingegeben werden, um ein Kennzeichnungsmodell zu erstellen.

## Claims

1. Method for computing a pair of digital data items representative of an emotional state, consisting in:
   acquiring:

   - a first series of physiological signals by at least one GSR and/or EDA electrodermal sensor
   - a second series of PPG physiological signals by a heart rate sensor
   - transmitting to a remote server said time-stamped signals as well as an identifier of the acquisition equipment
   - performing processing of each of said signals to characterize a data pair $S_{arousal}$, $S_{valence}$
   - said processing of said first series of signals being of the empirical mode decomposition (EMD) type over a sliding time window, the result of which provides the first value $S_{arousal}$ of said pair
   - said processing of said second series of signals comprising a step of band-pass filtering of frequencies between 0.04 and 0.26 Hz and of detecting peaks and RR inter-peak time measurement, over said sliding time window, the result of which provides the value $S_{valence}$ of said pair.

2. Method for computing a pair of digital data items representative of an emotional state according to claim 1, **characterized in that** said series of GSR and PPG data are timestamped and transmitted in the form of digital messages to a computer performing the computations for the real-time computing of the values $S_{arousal}$, $S_{valence}$.

3. Method for computing a pair of digital data items representative of an emotional state according to claim 1, **characterized in that** said series of GSR and PPG data are stored in computing buffers, including buffer memories for the application of sliding time window processing.

4. Method for computing a pair of digital data items representative of an emotional state according to claim 1, **char-**

**acterized in that** it comprises a step of band-pass filtering of said GSR signal at a bandwidth between 0.05 and 1 Hz.

5. Method for computing a pair of digital data items representative of an emotional state according to the preceding claim, **characterized in that** the processing for the determination of said signal $S_{Arousal}$ is carried out across a time window of 15 to 25 seconds, starting from the normalized spectral power of the GSR signal computed on the 0.045-0.25 Hz band.

6. Method for computing a pair of digital data items representative of an emotional state according to claim 1, **characterized in that** it comprises a step of band-pass filtering of said PPG signal at a bandwidth between 0.5 and 5 Hz.

7. Method for computing a pair of digital data items representative of an emotional state according to claim 1, **characterized in that** it comprises a viewing step comprising controlling the display of a graphic shape of which a first parameter is a function of the value of said signal $S_{arousal}$ and of which a second parameter is a function of the value of said signal $S_{valence}$.

8. Method for computing a pair of digital data items representative of an emotional state according to the preceding claim, **characterized in that** said first parameter comprises the size, thickness of the contour or shape factor, and **in that** said second parameter comprises the color and orientation of a main axis of said graphic shape.

9. Method for computing a pair of digital data items representative of an emotional state according to claim **1, characterized in that** it comprises a prior step of supervised learning consisting in presenting, to a panel of persons equipped with a device for acquiring said GSR and/or EDA and PPG physiological signals, a plurality of experimental plans formed by a succession of video sequences each associated with a digital descriptor ID (t), and recording the pairs of signals $S_{arousal}$ and $S_{valence}$ and their evolution over time, for each of the members of the panel, then injecting the structured data ($S_{arousal}$ and $S_{valence}$ (t); ID (t)) into a neural network to develop a characterization model.

[Fig 1]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- KR 20160085577
- US 2008214903 A
- US 2019239795 A
- US 2016345060 A

**Littérature non-brevet citée dans la description**

- **J.A.DOMÍNGUEZ-JIMÉNEZ ; K.C.CAMPO-LANDINES ; J.C.MARTINEZ-SANTOS ; E.J.DELAHOZ ; S.H.CONTRERAS-ORTIZ.** A machine learning model for emotion récognition from physiological signals. Universidad Tecnológica de Bolívar, 06 Décembre 2018

- **NEIDE SIMOES-CAPELA ; GIUSEPPINA SCHIAVORIE ; AMIT ACHARYYA ; WALTER DE RAEDT ; CHRIS VAN HOOF.** A data driven empirical itérative algorithm for GSR signal pre-processing auteurs Arvind Gautam. *2018 26th European Signal Processing Conférence (EUSIPCO),* 03 Septembre 2018, 1162-1166